# EUROPEAN PATENT SPECIFICATION

(11) **EP 4 319 865 B1**
(45) Date of publication and mention of the grant of the patent: **24.06.2026**
(21) Application number: 22717494.3
(22) Date of filing: 30.03.2022
(51) Int. Cl.: A61N 1/375, A61N 1/362, A61N 1/36, A61N 1/39, A61B 5/29, A61B 5/00, A61M 60/178, A61M 60/876, A61M 60/878, A61M 60/216

(54) **IMPLANTABLE MEDICAL DEVICE AND SYSTEM INCLUDING SAME**
IMPLANTIERBARE MEDIZINISCHE VORRICHTUNG UND SYSTEM DAMIT
DISPOSITIF MÉDICAL IMPLANTABLE ET SYSTÈME COMPRENANT CELUI-CI

(30) Priority: 08.04.2021 US 202163172234 P; 29.03.2022 US 202217706850
(43) Date of publication of application: 14.02.2024
(73) Proprietor: Medtronic, Inc., Minneapolis, Minnesota 55432 (US)
(72) Inventor: MERTZ, John, Minneapolis, Minnesota 55432 (US); MOTHILAL, Kamal Deep, Minneapolis, Minnesota 55432 (US); LYU, Suping, Minneapolis, Minnesota 55432 (US)
(74) Representative: Zimmermann & Partner Patentanwälte mbB
(86) International application number: PCT/US2022/022435
(87) International publication number: WO 2022/216484

(56) References cited:
- US-A- 5 336 246
- US-A- 5 431 695
- US-A- 5 456 698
- US-A1- 2014 277 234
- US-A1- 2019 290 911

## Description

### TECHNICAL FIELD

This disclosure generally relates to an implantable medical device and in particular to an implantable medical device system that includes the implantable medical device.

### BACKGROUND

Implantable medical device systems such as implantable pacemakers can deliver pacing pulses to a patient's heart and monitor conditions of the patient's heart. In some examples, the implantable pacemaker includes an electronic assembly disposed within a housing and one or more electrical leads. The electronic assembly may, for example, be implanted in a small pocket in the patient's chest. The electrical leads can be coupled to the electronic assembly, which may include one or more electronic components that generate pacing pulses and/or sense cardiac electrical activity. The electrical leads may extend from the electronic assembly to a target site (e.g., an atrium and/or a ventricle) such that electrodes disposed on or within the electrical leads are positioned at the target site. The electronic assembly may provide electrical stimulation to the target site and/or monitor cardiac electrical activity at the target site via the electrodes.

Document US 5,456,698 A relates to an enclosure for use in pacemaker as well as other implantable medical devices that has a substantially planar lid which is welded to a circumferential flange formed on a deep drawn shield, wherein the shield and lid form a "container" which is encased in a compliant shroud which isolates the welded flange from direct body contact and which enhances the biocompatibility of the entire device.

### SUMMARY

The techniques of this disclosure generally relate to an implantable medical device and an implantable medical device system that includes such device. The implantable medical device can include a sealed container that is disposed within a housing of the device. An electronic assembly and a battery can be disposed within the sealed container. The battery can be electrically connected to the electronic assembly. At least one of the electronic assembly or battery can be electrically connected to one or more components or devices disposed outside of the sealed container by one or more conductors that extend between the electronic assembly and such components or devices, where the conductors extend through the sealed container.

In one example, aspects of this disclosure relate to an implantable medical device having a housing that includes a polymeric material, a sealed container disposed within the housing, and an electronic assembly disposed within the container. The device also includes a battery disposed within the container and electrically connected to the electronic assembly.

In another example, aspects of this disclosure relate to an implantable medical device system that includes an implantable medical device and a lead adapted to be electrically connected to the implantable medical device. The implantable medical device includes a housing having a chamber and a lead receptacle, where the housing includes a polymeric material; a sealed container disposed within the chamber of the housing; and an electronic assembly disposed within the container. The device further includes a battery disposed within the container and electrically connected to the electronic assembly, and a conductor electrically connected to the electronic assembly and a contact disposed within the lead receptacle, where the conductor extends through the container.

In another, however non-claimed example, aspects of this disclosure related to a method of forming an implantable medical device system. The method includes disposing an electronic assembly and a battery within a container, where the battery is electrically connected to the electronic assembly; sealing the container; and disposing the container within a housing, where the housing includes a polymeric material. The method further includes electrically connecting the electronic assembly to a lead utilizing a conductor that extends from the electronic assembly and through the container to a lead receptacle of the polymeric housing, where a portion of the lead is disposed within the lead receptacle.

The details of one or more aspects of the disclosure are set forth in the accompanying drawings and the description below. Other features, objects, and advantages of the techniques described in this disclosure will be apparent from the description and drawings, and from the claims.

### BRIEF DESCRIPTION OF DRAWINGS

FIG. 1 is a schematic cross-section view of one embodiment of an implantable medical device.
FIG. 2 is a schematic perspective cross-section view of a housing of the implantable medical device of FIG. 1.
FIG. 3 is a schematic plan cross-section view of a sealed container of the implantable medical device of FIG. 1.
FIG. 4 is a schematic cross-section view of a portion of another embodiment of a sealed container.
FIG. 5 is a schematic plan view of another embodiment of a sealed container with a cover film of the sealed container partially removed from a tray of the sealed container.
FIG. 6 is a schematic cross-section view of one embodiment of an implantable medical device system that includes the implantable medical device of FIG. 1 and a lead.
FIG. 7 is a schematic cross-section view of another embodiment of an implantable medical device system.
FIG. 8 is a flowchart of one embodiment of a method of making the implantable medical device of FIG. 1.

### DETAILED DESCRIPTION

The techniques of this disclosure generally relate to an implantable medical device and an implantable medical device system that includes such device. The implantable medical device can include a sealed container that is disposed within a housing of the device. An electronic assembly and a battery can be disposed within the sealed container. The battery can be electrically connected to the electronic assembly. At least one of the electronic assembly or battery can be electrically connected to one or more components or devices disposed outside of the sealed container by one or more conductors that extend between the electronic assembly and such components or devices, where the conductors extend through the sealed container.

Implantable medical devices such as pacemakers can be disposed within a body of patient and provide various types of treatments to the patient through delivery of electric or other types of signals. Because it is disposed within the body, these devices are exposed to fluids or tissue that may be directed into a housing of the device and potentially damage or contaminate electronics or power sources disposed within the housing.

To prevent ingress of these fluids, the housing can be hermetically sealed; however, certain types of materials such as titanium or other metals may be required to form such hermetically sealed housings. These materials can be expensive, and techniques that can be utilized to seal these materials together can also be expensive and present various technical challenges. Further, in circumstances where an implantable medical device may be for short term use, expensive materials such as titanium may not be desirable.

One or more embodiments of implantable medical devices described herein may include a housing that includes one or more polymeric materials that may be less expensive than materials that are typically used for such devices. Such polymeric housings may not need to provide a hermetically sealed enclosure as electronic devices and power sources of the device can disposed within a sealed container that is disposed within the housing. The sealed container can prevent ingress of fluids or other contaminants that can damage electronic components or devices disposed within the housing. Further, various materials can be selected for the sealed container that may provide additional benefits such as shielding electronic components or devices from electromagnetic interference (EMI).

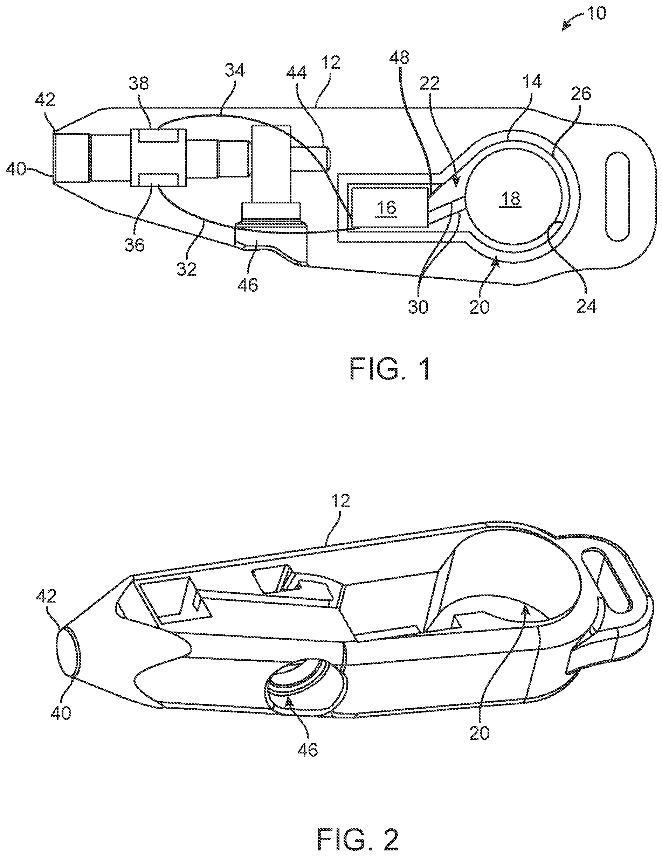

FIGS. 1-3 are various views of one embodiment of an implantable medical device 10. The device 10 has a housing 12 that includes a polymeric material, a sealed container 14 disposed within the housing, and an electronic assembly 16 disposed within the container. The device 10 also includes a battery 18 disposed within the container 14 that is electrically connected to the electronic assembly 16.

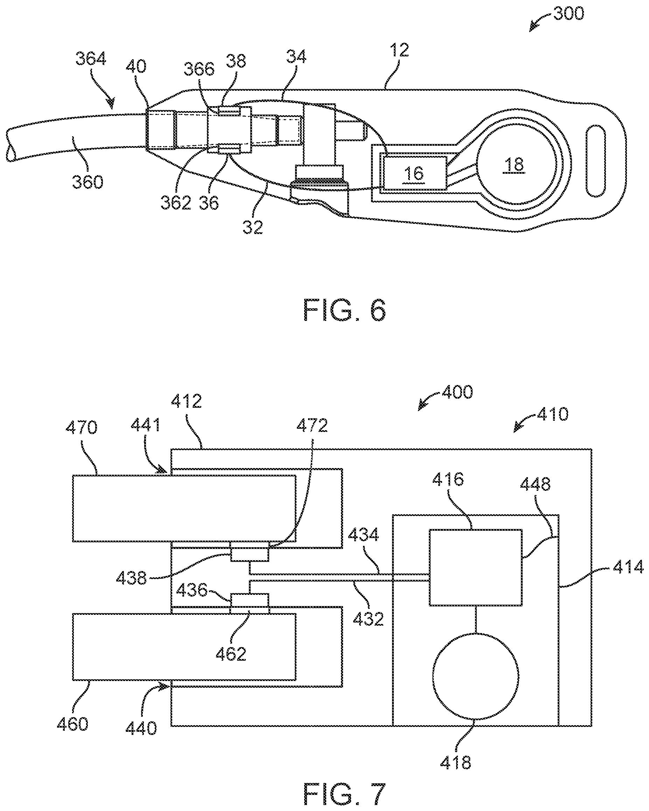

The implantable medical device 10 can be any suitable device that is adapted to be implanted within a body of a patient. In one or more embodiments, the device 10 can be a pacemaker. Further, in one or more embodiments, the device 10 can be a leadless cardiac monitor. The device 10 can include any other suitable medical devices such as a defibrillator, LVAD, neurostimulator, drug pump, etc. In one or more embodiments, the device 10 can be included in any suitable system (e.g., implantable medical device system 300 of FIG. 6). In one or more embodiments, the implantable medical device 10 can be included in a system that also includes one or more leads that are electrically connected to the electronic assembly 16 as is further described herein.

The housing 12 of the device 10 can take any suitable shape or shapes and have any suitable dimensions. Further, the housing 12 can include any suitable material or materials, e.g., metallic, polymeric, or inorganic materials. Suitable materials for the housing 12 can include at least one of titanium (e.g., any suitable grade such as grade 5 titanium), stainless steel, polymer, ceramic, glass, or combinations thereof such as laminates, composites, or miscible blends or mixtures. In one or more embodiments, the housing 12 can include any suitable polymeric material or materials, e.g., at least one of epoxy, polyurethane, silicone, polyolefin, acrylic polymer, polyester, polyetheletherketone, polysulfone, polymethylene oxide, or polyvinyl, or combinations thereof.

The housing 12 can be a unitary housing. In one or more embodiments, the housing 12 can include two or more portions that are connected using any suitable technique or techniques, e.g., welding, mechanically fastening, adhering, thermal bonding, diffusion bonding, laser-assisted diffusion bonding, solvent bonding, etc. Further, the housing 12 can be formed using any suitable technique or techniques, e.g., molding, thermoforming, laminating, over-molding, casting, insert molding, etc.

The housing 12 can include any suitable ports or receptacles that can connect the device to external components or systems. For example, the housing 12 can include a lead receptacle 40 disposed in any suitable portion or portions of the housing. As shown in FIG. 1, the lead receptacle 40 extends between a first end 42 and a second end 44 of the receptacle. The lead receptacle 40 can take any suitable shape or shapes and have any suitable dimensions. Housing 12 can also include a setscrew bore 46 disposed in any suitable portion or portions of the housing. The setscrew bore 46 can take any suitable shape or shapes and have any suitable dimensions.

Disposed within the housing 12 is the container 14. The container 14 can be disposed within any suitable portion or portions of the housing 12. As shown in FIG. 1, the container 14 is disposed within a chamber 20 of the housing 12. The chamber 20 can take any suitable shape or shapes and have any suitable dimensions. Although depicted as including a single container 14, the device 10 can include any suitable number of containers disposed within the housing 12.

The container 14 can take any suitable shape or shapes and have any suitable dimensions. The container includes an inner surface 24 and an outer surface 26. As used herein, the term "inner surface" means a surface of the container 14 that defines at least a portion of a cavity (e.g., cavity 22 of container 14 of FIGS. 1-3) within the container. Further, as used herein, the term "outer surface" means a surface of the container 14 that faces away from the cavity 22 within the container. The inner surface 24 of the container 14 defines a cavity 22 within the container.

The container 14 can include any suitable material or materials, e.g., at least one of a metallic, polymeric, or inorganic material. For example, the container 14 can include any suitable metallic material, e.g., at least one of titanium, stainless steel, gold, silver, tantalum, aluminum, nickel, cobalt, or chromium. Further, for example, the container 14 can include any suitable polymeric material, e.g., at least one of epoxy, polyolefin, nylon, polyester, polyamide, polyvinyl acetate, polyvinyl alcohol, acrylic polymer, methyl acrylic polymer, cellulose and its derivatives, polystyrene, Parylene, polyurethane, polysulfone, polyimide, polyetheretherketone, or liquid crystal polymer. In one or more embodiments, the container can include a polymer barrier film. Such polymer barrier film can include two or more layers to provide a multilayer polymer barrier film. Each layer of the multilayer polymer barrier film can include the same material or materials. In one or more embodiments, at least one layer of the multilayer polymer barrier film can include a material that is different from a material of one or more additional layers of the film. Further, in one or more embodiments, the polymer barrier film can include a thermoformed polymer barrier film that can be formed using any suitable thermoforming technique or techniques. In one or more embodiments, the container 14 can include one or more inorganic material or materials such as a ceramic material, e.g., at least one of ceramic, glass, or natural mineral. In general, the container can be formed using any suitable technique or techniques, e.g., at least one of thermoforming, vacuum forming, blow molding, or injection molding.

A wall of the container 14 can have any suitable thickness or thicknesses. Further, at least one of the thickness of the wall of the container 14 or the material properties of the container can be selected to have any suitable rigidity. In one or more embodiments, the container 14 can be complaint such that one or more portions of the container conform to one or more portions of the electronic assembly 16 and battery 18.

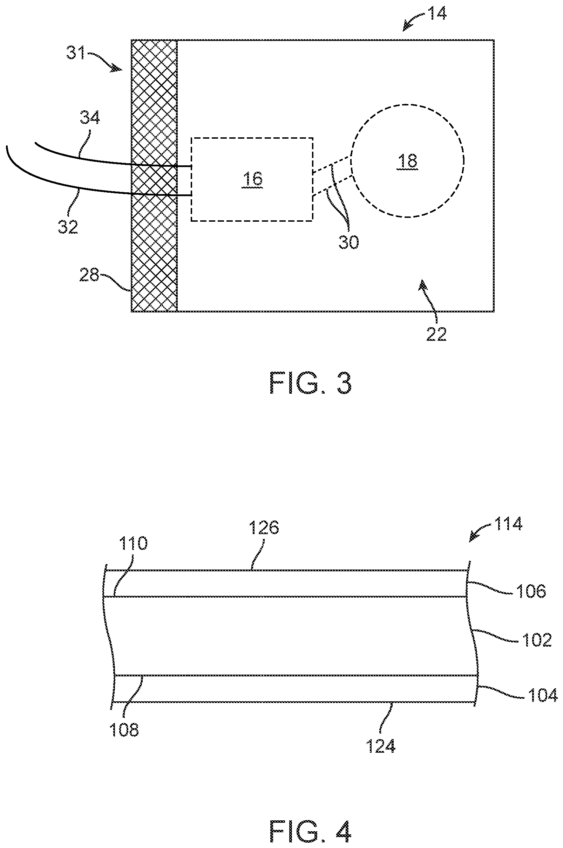

In one or more embodiments, the container 14 can include a metal foil. For example, FIG. 4 is a schematic cross-section view of a portion of a container 114. All of the design considerations and possibilities described herein regarding the container 14 of FIGS. 1-3 apply equally to the container 114 of FIG. 4. The container 114 includes a metal foil 102. The metal foil 102 can have any suitable dimensions. Further, the metal foil 102 can include any suitable material or materials, e.g., the same metallic materials describe herein regarding the container 14 of FIGS. 1-3. The metal foil 102 can also include any suitable number of metal layers. In one or more embodiments, the metal foil 102 can include one or more polymer layers disposed in any suitable relationship with the metal layer or layers.

In one or more embodiments, the container 114 can also include one or more polymer layers disposed on at least one of a first major surface 108 or a second major surface 110 of the metal foil 102. For example, the container 114 includes a first polymer layer 104 disposed on the first major surface 108 of the metal foil 102 and a second polymer layer 106 disposed on the second major surface 110 of the metal foil. The first polymer layer 104 can define an inner surface 124 of the container 114, and the second polymer layer 106 can define an outer surface 126 of the container.

Each polymer layer 104, 106 can include any suitable material or materials, e.g., at least one of epoxy, polyolefin, nylon, polyester, polyvinyl acetate, polyvinyl alcohol, acrylic polymer, methyl acrylic polymer, cellulose and its derivatives, polystyrene, Parylene, polyurethane, polysulfone, polyimide, polyetheretherketone, or liquid crystal polymer. Further, each polymer layer 104, 106 can include the same materials or different materials. The polymer layers 104, 106 can each include any suitable number of sublayers. In one or more embodiments, one or more additional polymer sublayers can be disposed between one or more additional metal foil layers to form the container 114.

Returning to FIGS. 1-3, the container 14 can be sealed using any suitable technique or techniques to provide a sealed container. For example, as shown in FIG. 3, the container 14 includes a seal 28 that can seal an opening 31 in the container. Any suitable technique or techniques can be utilized to form the seal 28, e.g., heat sealing, ultrasonic welding, mechanically fastening, welding, crimping, adhering, chemical curing, etc. The opening 31 can provide access to a cavity 22 within the housing 12 such that the electronic assembly 16, the battery 18, and any suitable additional elements or components can be disposed therein. The opening 31 can be disposed in any suitable portion or portions of the container 14. In one or more embodiments, the container 14 can include two or more openings that can be sealed using any suitable technique or techniques. In one or more embodiments, the container 14 can be a hermetically sealed container.

Disposed within the container 14 is the electronic assembly 16. The assembly 16 can include any discrete and/or integrated electronic circuit components that implement analog and/or digital circuits capable of producing the functions attributed to the implantable medical device 10 described herein. In one or more embodiments, the assembly 16 can also include components for sensing other physiological parameters, such as acceleration, pressure, sound, and/or impedance. The assembly 16 can include any suitable electronic component, e.g., at least one of a capacitor, transistor, integrated circuit, including controller or multiplexer, sensor, accelerometer, inductive charging coil, optical components such as emitters and detectors, etc. Although depicted as including one electronic assembly 16, the device 10 can include any suitable number of electronic assemblies. Further, the device 10 can include one or more additional electronic components or elements disposed outside of the container 14 and either within or on the housing 12.

The device 10 can also include a battery 18 disposed within the container 14. The battery 18 can include any suitable power source. Further, the battery 18 can be electrically connected to the electronic assembly 16 using any suitable technique or techniques. As shown in FIG. 1, the battery 18 is electrically connected to the electronic assembly 16 with conductors 30. Any suitable conductors 30 can be utilized to electrically connect the electronic assembly 16 to the battery 18.

The container 14 can also include any suitable additional components or compositions that provide various functionalities to the container. For example, a liquid or solid polymer can be disposed within the container 14. The liquid polymer can include any suitable material or materials, e.g., at least one of hydrocarbon fluid, silicone oil, fluoro liquid, motor oil, vegetable oil, hydrogenated vegetable oil, or low molecular weight polymer. Further, the solid polymer can include any suitable material or materials, e.g., at least one of an epoxy, phenolic polymer, silicone, foam, or polyolefin. Further, in one or more embodiments, a desiccant can be disposed within the container 14. The desiccant can include any suitable material or materials that can absorb moisture present within the container 14, e.g., a molecular sieve, silica gel, etc.

At least one of the electronic assembly 16 or the battery 18 can be electrically connected to one or more additional components or devices that are disposed outside of the container 14. For example, a conductor 32 can be electrically connected to the electronic assembly 16. The conductor 32 can extend through any suitable portion of the sealed container 14. In one or more embodiments, the conductor 32 can extend through the edge seal 28 of the sealed container 14 as shown in FIG. 3 such that the container seals around the conductor. In one or more embodiments, the conductor 32 can electrically connect the electronic assembly 16 to a second electronic assembly (not shown) disposed outside of the container 14 and either within or on the housing 12. Further, the device 10 can include any suitable number of conductors that extend from within the sealed container 14 to outside of the container. For example, as shown in FIGS. 1-3, a second conductor 34 is electrically connected to the electronic assembly 16 and extends through the sealed container 14, e.g., through edge seal 28 as shown in FIG. 3.

The conductor 32 can electrically connect the electronic assembly 16 to a contact 36 disposed within the housing 12. Further, the second conductor 34 can electrically connect the electronic assembly 16 or a second electronic assembly to a second contact 38 also disposed within the housing 12. The contacts 36, 38 can be disposed in a suitable location within the housing 12. In one or more embodiments, at least one of the contact 36 or the second contact 38 can be disposed external to the housing 12, e.g., on an outer surface of the housing. In one or more embodiments, at least one of the contacts 36, 38 can be disposed within the lead receptacle 40 such that electronic assembly 16 can be electrically connected to a lead (e.g., lead 360 of implantable medical device system 300 of FIG. 6) disposed within the lead receptacle as is further described herein.

The device 10 can also include a ground conductor 48 that electrically connects the electronic assembly 16 to the container 14. The ground conductor 48 can include any suitable conductor or conductors. In one or more embodiments, the ground conductor 48 can electrically connect the battery 18 to the container 14. The ground conductor 48 can provide a ground path between at least one of the electronic assembly 16 or the battery 18 and the container 14.

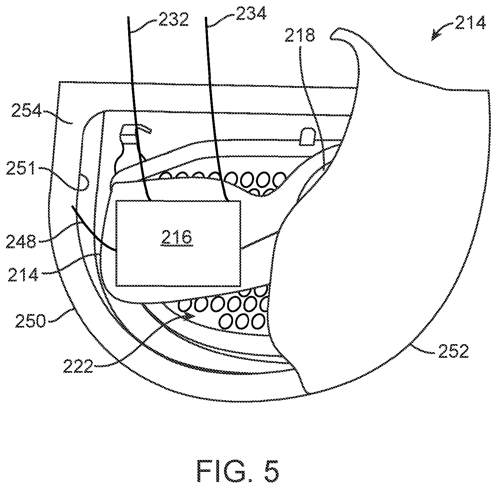

The device 10 can include any suitable container 14. For example, FIG. 5 is a schematic plan view of a container 214. All of the design considerations and possibilities described herein regarding the container 14 of FIGS. 1-3 apply equally to the container 214 of FIG. 5. The container 214 includes a tray 250 and a cover film 252 sealed to the tray. The cover film 252 is shown as being partially removed from the tray 250 in FIG. 5 for descriptive purposes; however, the cover film can be completely sealed to the tray to provide a sealed cavity 222 within the container 214. The cover film 252 can be sealed to the tray 250 using any suitable technique or techniques, e.g., heat sealing, ultrasonic welding, mechanically fastening, welding, crimping, adhering, chemical curing, etc.

The tray 250 can take any suitable shape or shapes and having any suitable dimensions. Further, the tray 250 can include any suitable material or materials, e.g., the same materials described herein regarding the container 14 of device 10 of FIGS. 1-3. Further, the tray 250 can be formed using any suitable technique or techniques, e.g., the same techniques described herein regarding the container 14. In one or more embodiments, the tray 250 can include an edge 254 that can provide a surface for sealing the cover film 252 to the tray.

The cover film 252 can include any suitable material or materials, e.g., the same materials described herein regarding the container 14 of FIGS. 1-3. Further, the cover film 252 can include the same materials as the tray 250. In one or more embodiments, the cover film 252 can include one or more materials that different from the materials utilized for form the tray 250. In one or more embodiments, the tray 250 can be manufactured such that it has greater rigidity than that of the cover film 252. Further, in one or more embodiments, the cover film 252 can be manufactured such that it has greater rigidity than that of the tray 250.

One or more conductors 232, 234 can be electrically connected to electronic assembly 216 disposed within the cavity 222 of the container 214. The conductors 232, 234 can electrically connect at least one of the electronic assembly 216 or battery 218 to one or more components or devices disposed outside of the container 214. The conductors 232, 234 can extend over the edge 254 of the tray 250. And the cover film 252 can be sealed to the tray 250 such that the conductors 232, 234 extend from the container 214 without providing fluid ingress into the container. Further, the electronic assembly 216 can be grounded to the tray 250 by ground conductor 248. Such ground conductor 248 can extend between an inner surface 251 of the tray 250 and the electronic assembly 216 such the ground conductor does not extend between the edge 254 of the tray and the cover film 252.

The various embodiments of implantable medical devices described herein can be utilized in any suitable system. For example, FIG. 6 is a schematic cross-section view of one embodiment of an implantable medical device system to 300. The system 300 includes the implantable medical device 10 of FIGS. 1-3 and a lead 360 that is adapted to be electrically connected to the implantable medical device. The lead 360 can include any suitable lead. In one or more embodiments, the lead 360 includes a lead contact 362 that is adapted to electrically connect the lead to the contact 36 disposed within the lead receptacle 40 when a portion 364 of the lead is disposed within the lead receptacle. In one or more embodiments, the lead 360 can include a second lead contact 366 that is adapted to electrically connect the lead to the second contact 38 disposed in the lead receptacle 40 when the portion 364 of the lead is disposed within the lead receptacle.

The system 300 can include any suitable number of leads electrically connected to the electronic assembly 16 disposed within the housing 12 of the implantable medical device 10. For example, FIG. 7 is a schematic cross-section view of another embodiment of an implantable medical device system 400. All of the design considerations and possibilities described herein regarding the implantable medical device system 300 of FIG. 6 apply equally to the implantable medical device system 400 of FIG. 7.

One difference between the system 400 of FIG. 7 and system 300 FIG. 6 is that the system 400 includes a first lead 460 and a second lead 470. The first lead 460 is disposed within a lead receptacle 440 of housing 412 of the device 410, and the second lead 470 is disposed within a second lead receptacle 441 of the housing 412.The first and second leads 460, 470 can include any suitable lead, e.g., lead 360 of system 300 of FIG. 6. The first lead 460 can include a lead contact 462 that is electrically connected to contact 436 of the device 410. The lead context 462 and the contact 436 electrically connect the lead 460 to electronic assembly 416 of the device 410 via conductor 432. The conductor 432 extends through sealed container 414, which contains electronic assembly 416 and battery 418. Further, the second lead 470 can include a second lead contact 472 that is adapted to be electrically connected to second contact 438 of the device 410. The second lead contact 472 of the second lead 470 and the second contact 438 electrically connect the second lead to the electronic assembly 416 via second conductor 434. The second conductor 434 extends through the sealed container 414. In one or more embodiments, a ground conductor 448 can be electrically connected to the electronic assembly 416 and the container 414 to provide a ground path for at least one of the electronic assembly or the battery 418.

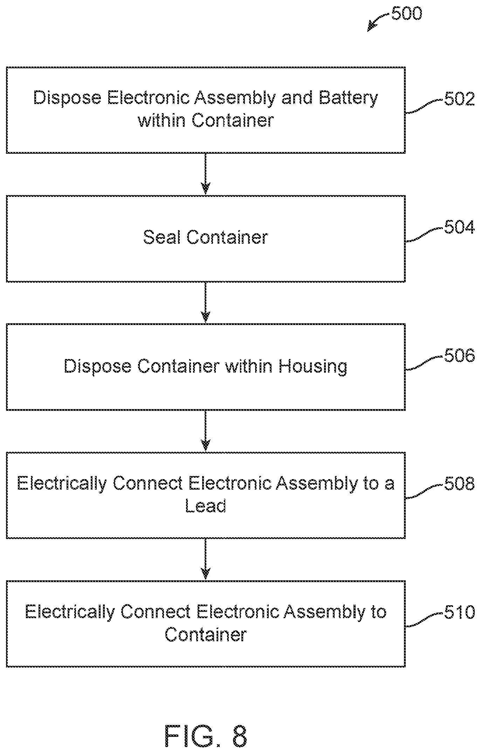

Any suitable technique or techniques can be utilized to manufacture the various embodiments of implantable medical devices and systems described herein. For example, FIG. 8 is a flowchart of one embodiment of a method of forming an implantable medical device system 400. Although described in regard to system 400 of FIG. 7, the method 500 can be utilized to form any suitable implantable medical device system.

At 502, the electronic assembly 416 and the battery 418 can be disposed within the container 414 using any suitable technique or techniques. In one or more embodiments, the container 414 can be formed around the electronic assembly 416 and the battery 418 such that the assembly and battery are disposed within the container. For example, the electronic assembly 416 and the battery 418 can be disposed on a sheet of material or materials utilized to form the container 414, and the sheet can be formed around the assembly and the battery to provide the container. The container 414 can be formed prior to or after disposing the electronic assembly 416 and the battery 418 in the container. In one or more embodiments, the container 414 can be partially formed prior to disposing the electronic assembly 416 and the battery 418 within the container, and then the container can be completely formed after the electronic assembly and the battery are disposed within the partially formed container. Although not shown, a desiccant or other composition can be disposed within the container 414 prior to sealing the container.

At 504, the container 414 can be sealed using any suitable technique or techniques. In one or more embodiments, the container 414 can be hermetically sealed. In one or more embodiments, an edge of the container 414 can be sealed to provide an edge seal (e.g., edge seal 28 of FIG. 3). The container 414 can be edge-sealed such that the conductors 432, 434 extend through the edge seal.

The container 414 can be disposed within the housing 412 at 506 using any suitable technique or techniques. In embodiments where the housing 412 includes one or more portions, the container 414 can be disposed within or on a first portion of the housing, and a second or additional portions of the housing can be connected to the first portion using any suitable technique or techniques such that the container is disposed within the housing.

At 508, the electronic assembly 416 can be electrically connected to the lead 460 utilizing the conductor 432 that extends from the electronic assembly and through the container 414 to the lead receptacle 440 of the polymeric housing 412 when a portion of the lead is disposed within the receptacle 440. In one or more embodiments, the electronic assembly 416 can be electrically connected to the second lead 470 utilizing the second conductor 434 that extends from the electronic assembly and through the container 414 to the second lead receptacle 441 of the housing 412 when a portion of the second lead is disposed within the second lead receptacle. The electronic assembly 416 can be connected to the container 414 by the ground conductor 448 at 510.

It should be understood that various aspects disclosed herein may be combined in different combinations than the combinations specifically presented in the description and accompanying drawings. It should also be understood that, depending on the example, certain acts or events of any of the processes or methods described herein may be performed in a different sequence, may be added, merged, or left out altogether (e.g., all described acts or events may not be necessary to carry out the techniques). In addition, while certain aspects of this disclosure are described as being performed by a single module or unit for purposes of clarity, it should be understood that the techniques of this disclosure may be performed by a combination of units or modules associated with, for example, a medical device.

In one or more examples, the described techniques may be implemented in hardware, software, firmware, or any combination thereof. If implemented in software, the functions may be stored as one or more instructions or code on a computer-readable medium and executed by a hardware-based processing unit. Computer-readable media may include computer-readable storage media, which corresponds to a tangible medium such as data storage media (e.g., RAM, ROM, EEPROM, flash memory, or any other medium that can be used to store desired program code in the form of instructions or data structures and that can be accessed by a computer).

Instructions may be executed by one or more processors, such as one or more digital signal processors (DSPs), general purpose microprocessors, application specific integrated circuits (ASICs), field programmable logic arrays (FPGAs), or other equivalent integrated or discrete logic circuitry. Accordingly, the term "processor" as used herein may refer to any of the foregoing structure or any other physical structure suitable for implementation of the described techniques. Also, the techniques could be fully implemented in one or more circuits or logic elements.

## Claims

1. An implantable medical device (10) comprising:
a housing (12) comprising a polymeric material;
a sealed container (14) disposed within the housing (12);
an electronic assembly (16) disposed within the container (14); and
a battery (18) disposed within the container (14) and electrically connected to the electronic assembly (16),
wherein the container (14) comprises a polymeric material.

2. The device (10) of claim 1, further comprising a conductor (32) electrically connected to the electronic assembly (16) and extending through the sealed container (14).

3. The device (10) of claim 2, wherein the conductor (32) extends through an edge seal (28) of the sealed container (14).

4. The device (10) of claim 1, further comprising a ground conductor (48) electrically connected to the electronic assembly (16) and the container (14).

5. The device (10) of any one of claims 1-4, wherein the container (14) comprises a metallic material.

6. The device (10) of any one of claims 1-4, wherein the container (14) comprises a polymer barrier film.

7. The device of any one of claims 1-4, wherein the container (14) comprises a tray (250) and a cover film (252) sealed to the tray.

8. The device (10) of any one of claims 1-4, wherein the container (14) comprises a hermetically sealed container (14).

9. An implantable medical device system comprising an implantable medical device (10) according to claim 1 and a lead adapted to be electrically connected to the implantable medical device (10), the housing (12) of the implantable medical device (10) further comprises a chamber and a lead receptacle and the implantable medical device (10) further comprises a conductor (32) electrically connected to the electronic assembly (16) and a contact disposed within the lead receptacle, wherein the conductor (32) extends through the container (14).

10. The system of claim 9, wherein the lead comprises a lead contact that is adapted to electrically connect the lead to the contact of the lead receptacle when a portion of the lead is disposed within the lead receptacle.

11. The system of claim 9, wherein the conductor (32) extends through an edge seal of the sealed container (14).

12. The system of claim 9, further comprising a ground conductor (48) electrically connected to the electronic assembly (16) and the container (14).

13. The system of any one of clams 9-12, wherein the container (14) comprises a metal foil.

14. The system of claim 13, wherein the metal foil comprises a polymer layer disposed on an outer surface of the metal foil.

15. The system of claim 14, wherein the polymer layer comprises at least one of epoxy, polyolefin, polyamide, polyester, polyvinyl acetate, polyvinyl alcohol, acrylic polymer, methyl acrylic polymers, cellulose and its derivatives, polystyrene, Parylene, polyurethane, polysulfone, polyimide, polyetheretherketone, or liquid crystal polymer.

## Patentansprüche

1. Implantierbare medizinische Vorrichtung (10) umfassend:
ein Gehäuse (12), das ein Polymermaterial umfasst;
einen abgedichteten Behälter (14), der in dem Gehäuse (12) angeordnet ist;
eine Elektronikbaugruppe (16), die in dem Behälter (14) angeordnet ist; und
eine Batterie (18), die in dem Behälter (14) angeordnet und elektrisch mit der Elektronikbaugruppe (16) verbunden ist,
wobei der Behälter (14) ein Polymermaterial umfasst.

2. Vorrichtung (10) nach Anspruch 1, ferner umfassend einen Leiter (32), der elektrisch mit der Elektronikbaugruppe (16) verbunden ist und durch den abgedichteten Behälter (14) verläuft.

3. Vorrichtung (10) nach Anspruch 2, wobei der Leiter (32) durch eine Randdichtung (28) des abgedichteten Behälters (14) verläuft.

4. Vorrichtung (10) nach Anspruch 1, ferner umfassend einen Masseleiter (48), der elektrisch mit der Elektronikbaugruppe (16) und dem Behälter (14) verbunden ist.

5. Vorrichtung (10) nach einem der Ansprüche 1-4, wobei der Behälter (14) ein metallisches Material umfasst.

6. Vorrichtung (10) nach einem der Ansprüche 1-4, wobei der Behälter (14) einen Polymerbarrierefilm umfasst.

7. Vorrichtung nach einem der Ansprüche 1-4, wobei der Behälter (14) eine Schale (250) und eine Abdeckfolie (252), die an die Schale gesiegelt ist, umfasst.

8. Vorrichtung (10) nach einem der Ansprüche 1-4, wobei der Behälter (14) einen hermetisch abgedichteten Behälter (14) umfasst.

9. Implantierbares medizinisches Vorrichtungssystem, umfassend eine implantierbare medizinische Vorrichtung (10) nach Anspruch 1 und eine Leitung, die dafür ausgelegt ist, elektrisch mit der implantierbaren medizinischen Vorrichtung (10) verbunden zu werden, wobei das Gehäuse (12) der implantierbaren medizinischen Vorrichtung (10) ferner eine Kammer und eine Leitungsaufnahme umfasst und die implantierbare medizinische Vorrichtung (10) ferner einen Leiter (32), der elektrisch mit der Elektronikbaugruppe (16) verbunden ist, und einen Kontakt, der in der Leitungsaufnahme angeordnet ist, umfasst, wobei der Leiter (32) durch den Behälter (14) verläuft.

10. System nach Anspruch 9, wobei die Leitung einen Leitungskontakt umfasst, der dafür ausgelegt ist, die Leitung elektrisch mit dem Kontakt der Leitungsaufnahme zu verbinden, wenn ein Teil der Leitung in der Leitungsaufnahme angeordnet ist.

11. System nach Anspruch 9, wobei der Leiter (32) durch eine Randdichtung des abgedichteten Behälters (14) verläuft.

12. System nach Anspruch 9, ferner umfassend einen Masseleiter (48), der elektrisch mit der Elektronikbaugruppe (16) und dem Behälter (14) verbunden ist.

13. System nach einem der Ansprüche 9-12, wobei der Behälter (14) eine Metallfolie umfasst.

14. System nach Anspruch 13, wobei die Metallfolie eine Polymerschicht umfasst, die an einer Außenfläche der Metallfolie angeordnet ist.

15. System nach Anspruch 14, wobei die Polymerschicht wenigstens eines von Epoxy, Polyolefin, Polyamid, Polyester, Polyvinylacetat, Polyvinylalkohol, Acrylpolymer, Methylacrylpolymere, Cellulose und ihre Derivate, Polystyrol, Parylen, Polyurethan, Polysulfon, Polyimid, Polyetheretherketon oder Flüssigkristallpolymer umfasst.

## Revendications

1. Dispositif médical implantable (10) comprenant :
un boîtier (12) comprenant une matière polymère,
un récipient scellé (14) disposé à l'intérieur du boîtier (12) ;
un ensemble électronique (16) disposé à l'intérieur du récipient (14) ; et
une batterie (18) disposée à l'intérieur du récipient (14) et connectée électriquement à l'ensemble électronique (16),
le récipient (14) comprenant un matériau polymère.

2. Dispositif (10) selon la revendication 1, comprenant en outre un conducteur (32) connecté électriquement à l'ensemble électronique (16) et s'étendant à travers le récipient scellé (14).

3. Dispositif (10) selon la revendication 2, le conducteur (32) s'étendant à travers un joint d'étanchéité de bord (28) du récipient scellé (14).

4. Dispositif (10) selon la revendication 1, comprenant en outre un conducteur de masse (48) connecté électriquement à l'ensemble électronique (16) et au récipient (14).

5. Dispositif (10) selon l'une quelconque des revendications 1 à 4, le récipient (14) comprenant un matériau métallique.

6. Dispositif (10) selon l'une quelconque des revendications 1 à 4, le récipient (14) comprenant un film barrière polymère.

7. Dispositif selon l'une quelconque des revendications 1 à 4, le récipient (14) comprenant un plateau (250) et un film de recouvrement (252) scellé au plateau.

8. Dispositif (10) selon l'une quelconque des revendications 1 à 4, le récipient (14) comprenant un récipient hermétiquement scellé (14).

9. Système de dispositif médical implantable comprenant un dispositif médical implantable (10) selon la revendication 1 et un fil adapté pour être connecté électriquement au dispositif médical implantable (10), le boîtier (12) du dispositif médical implantable (10) comprenant en outre une chambre et un réceptacle de fil et le dispositif médical implantable (10) comprenant en outre un conducteur (32) connecté électriquement à l'ensemble électronique (16) et un contact disposé à l'intérieur du réceptacle de fil, le conducteur (32) s'étendant à travers le récipient (14).

10. Système selon la revendication 9, le fil comprenant un contact de fil qui est adapté pour connecter électriquement le fil au contact du réceptacle de fil lorsqu'une partie du fil est disposée à l'intérieur du réceptacle de fil.

11. Système selon la revendication 9, le conducteur (32) s'étendant à travers un joint de bord du récipient scellé (14).

12. Système selon la revendication 9, comprenant en outre un conducteur de masse (48) connecté électriquement à l'ensemble électronique (16) et au récipient (14).

13. Système selon l'une quelconque des revendications 9 à 12, le récipient (14) comprenant une feuille métallique.

14. Système selon la revendication 13, la feuille métallique comprenant une couche de polymère disposée sur une surface extérieure de la feuille métallique.

15. Système selon la revendication 14, la couche de polymère comprenant au moins un matériau parmi les matériaux suivants : époxy, polyoléfine, polyamide, polyester, poly(acétate de vinyle), alcool polyvinylique, polymère acrylique, polymères méthylacryliques, cellulose et ses dérivés, polystyrène, parylène, polyuréthane, polysulfone, polyimide, polyétheréthercétone, ou polymère à cristaux liquides.
